# EUROPEAN PATENT APPLICATION

(11) **EP 2 902 059 A1**
(43) Date of publication of application: **05.08.2015**
(21) Application number: 12885764.6
(22) Date of filing: 27.09.2012
(51) Int. Cl.: A61M 5/28, A61J 3/00

(54) **BARREL FOR PRE-FILLED SYRINGE, PUNCTURE DEVICE FOR PRE-FILLED SYRINGE, PRE-FILLED SYRINGE AND BARREL PACKAGING BODY FOR PRE-FILLED SYRINGE**

(71) Applicant: Terumo Kabushiki Kaisha, Tokyo 151-0072 (JP)
(72) Inventor: OKIHARA, Hitoshi, Fujinomiya-shi Shizuoka 418-0004 (JP); TAKEMOTO, Masafumi, Fujinomiya-shi Shizuoka 418-0004 (JP)
(74) Representative: Casalonga
(86) International application number: PCT/JP2012/074909
(87) International publication number: WO 2014/049781

(57) **Abstract**

An outer cylinder 2 for a prefilled syringe is mountable a piercing member 6 which includes a piercing member body 60 having a piercing member side engaging portion 62 and a hollow needle 61 having a needle portion at both ends thereof and mounted to the piercing member body 60. The outer cylinder 2 for the prefilled syringe has an outer cylinder body 4 which has a distal part 41 having an opening 48 and an outer cylinder closure member 5 which seals the opening 48 of the distal part 41, accommodates a sealing member 10 through which the needle portion 61b of the hollow needle 61 is penetrable, and is mounted on the distal part 41 of the outer cylinder body 4. The outer cylinder closure member 5 has a piercing member mounting portion 50 on which the piercing member 6 is mountable and a regulation portion which regulates the separation and rotation of the outer cylinder closure member 5 mounted on the outer cylinder body 4. The outer cylinder closure member 5 further includes an outer cylinder closure member side engaging portion 53 which engages the piercing member side engaging portion 62 when the outer cylinder 2 is pressed into the piercing member 6 from the side of the outer cylinder closure member 5 and which regulates the separation of the piercing member 6 from the outer cylinder closure member 5, after the outer cylinder closure member side engaging portion engages the piercing member side engaging portion 62.

## Description

### TECHNICAL FIELD

The present invention relates to an outer cylinder for a prefilled syringe, a piercing tool for the prefilled syringe, the prefilled syringe, and a packaging body for packaging the outer cylinder for the prefilled syringe.

### BACKGROUND ART

The prefilled syringe is provided with a medical liquid such as a liquid medicine and a medical agent solution filled therein. The front end of a certain type of a prefilled syringe is sealed with a cap on which a double-ended needle can be mounted. A piercing member having the double-ended needle is mounted on the cap when the prefilled syringe is used. An example of the piercing member having the double-ended needle and an example of a form of mounting the piercing member having the double-ended needle on the cap are disclosed in a patent document 1 (Japanese Unexamined Patent Application Publication No. 2010-540122, WO -2009/043000).

PATENT DOCUMENT 1: Japanese Unexamined Patent Application Publication No. 2010-540122, (WO -2009/043000)

### DISCLOSURE OF THE INVENTION

### PROBLEMS TO BE SOLVED BY THE INVENTION

In the prefilled syringe of the patent document 1, because the piercing member is mounted on the prefilled syringe by means of only the thread engagement between the piercing member and the outer cylinder, there is a possibility that when the prefilled syringe is used to administer a medical agent to a patient, the thread engagement loosens and the medical agent leaks.

It is an object of the present invention to provide an outer cylinder for a prefilled syringe, a piercing tool for the prefilled syringe, and the prefilled syringe in which it is easy to perform an operation of mounting a piercing member having a double-ended needle on an closure member of the syringe which seals a front end of the piercing member and prevent a medical agent from leaking because the closure member mounted on the outer cylinder for the syringe is so constructed that the closure member does not loosen in performing an operation of mounting the piercing member on the closure member, and a packaging body for packaging the outer cylinder for the prefilled syringe.

### MEANS FOR SOLVING THE PROBLEMS

In order to solve the above problem is the following.

An outer cylinder for a prefilled syringe on which a piercing member including a hollow needle having a needle portion at both ends thereof and a piercing member body on which said hollow needle is mounted and which has a piercing member side engaging portion, said outer cylinder for said prefilled syringe has an outer cylinder body which has a distal part having an opening and an outer cylinder closure member which seals said opening of said distal part, accommodates a sealing member through which said needle portion of said hollow needle is penetrable, and is mounted on said distal part of said outer cylinder body; said outer cylinder closure member has a piercing member mounting portion on which said piercing member is mountable and a regulation portion which regulates separation and rotation of said outer cylinder closure member mounted on said outer cylinder body; and said outer cylinder closure member further includes an outer cylinder closure member side engaging portion which engages said piercing member side engaging portion when said outer cylinder for said prefilled syringe is pressed into said piercing member from a side of said outer cylinder closure member and regulates separation of said piercing member from said outer cylinder closure member after said outer cylinder closure member side engaging portion engages said piercing member side engaging portion.

Also, in order to solve the above problem is the following.

A prefilled syringe comprises the above outer cylinder for a prefilled syringe, a gasket accommodated inside said outer cylinder and being liquid-tightly slidable inside said outer cylinder, and a medical agent accommodated inside a space formed of said outer cylinder and said gasket.

Also, in order to solve the above problem is the following.

A piercing tool for a prefilled syringe which can be mounted on the above outer cylinder for a prefilled syringe or on said outer cylinder closure member of the above prefilled syringe, said piercing tool has a hollow needle having said needle portion at both ends thereof; said piercing member on which said hollow needle is mounted and which is mountable on said outer cylinder closure member and has said piercing member body having said piercing member side engaging portion; and an accommodation member separably accommodating said piercing member; and said accommodation member is separated from said piercing member by pulling said accommodation member in a distal direction after said piercing member is mounted on said outer cylinder closure member, and said piercing member side engaging portion and said outer cylinder closure member side engaging portion engage each other.

Also, in order to solve the above problem is the following.

A packaging body for packaging outer cylinders for a prefilled syringe accommodates a plurality of the above outer cylinders for a prefilled syringe and can be subjected to or is subjected to autoclave sterilization, said packaging body comprises a container whose upper surface is open and which has shape retainability; an outer cylinder holding member is capable of holding a plurality of said outer cylinders for said prefilled syringe; a plurality of said outer cylinders for said prefilled syringe is held by said outer cylinder holding member; a sheet-shaped lid member airtightly seals said open upper surface of said container and is peelable from said container; and an air-permeable part, having bacteria impermeability and water vapor permeability, which is provided in said container or said lid member.

### BRIEF DESCRIPTION OF THE DRAWINGS

Fig. 1 is a front view of a prefilled syringe of the present invention and a piercing tool for the prefilled syringe.
Fig. 2 is an enlarged vertical sectional view of the prefilled syringe shown in Fig. 1.
Fig. 3 is an enlarged vertical sectional view of the piercing tool for the prefilled syringe shown in Fig. 1
Fig. 4 is a front view of an outer cylinder for the prefilled syringe of the present invention.
Fig. 5 is a right side view of the outer cylinder shown in Fig. 4.
Fig. 6 is an enlarged plan view of the outer cylinder shown in Fig. 4.
Fig. 7 is a perspective view of the outer cylinder shown in Fig. 4.
Fig. 8 is an enlarged front view of the outer cylinder for the prefilled syringe of the present invention and an outer cylinder closure member for the prefilled syringe.
Fig. 9 is a right side view of the outer cylinder closure member shown in Fig. 8.
Fig. 10 is a vertical sectional view of the outer cylinder closure member shown in Fig. 8.
Fig. 11 is a perspective view of the outer cylinder closure member shown in Fig. 8 as viewed from above.
Fig. 12 is a perspective view of the outer cylinder closure member shown in Fig. 8 as viewed from below.
Fig. 13 is an enlarged front view of a piercing member for use in the piercing tool for the prefilled syringe of the present invention.
Fig. 14 is a left side view of the piercing member shown in Fig. 13.
Fig. 15 is a vertical sectional view of the piercing member shown in Fig. 13.
Fig. 16 is a vertical sectional view of the piercing member shown in Fig. 14.
Fig. 17 is a perspective view of the piercing member shown in Fig. 13.
Fig. 18 is an enlarged front view of an accommodation member for the piercing tool for the prefilled syringe of the present invention.
Fig. 19 is a vertical sectional view of the accommodation member shown in Fig. 18.
Fig. 20 is an enlarged front view of a plunger for use in the prefilled syringe of the present invention and an syringe for the prefilled syringe.
Fig. 21 is a vertical sectional view of a gasket for use in the prefilled syringe of the present invention and the injector for the prefilled syringe.
Fig. 22 is an explanatory view for explaining constituent members for use in the prefilled syringe and the piercing member of the present invention.
Fig. 23 is an explanatory view for explaining the action of the prefilled syringe of the present invention and that of the piercing member.
Fig. 24 is an explanatory view for explaining the action of the prefilled syringe of the present invention and that of the piercing member.
Fig. 25 is an explanatory view for explaining the action of the prefilled syringe of the present invention and that of the piercing member.
Fig. 26 is an explanatory view for explaining the action of the prefilled syringe of the present invention and that of the piercing member.
Fig. 27 is a perspective view of a packaging body for packaging outer cylinders for the prefilled syringe of the present invention.
Fig. 28 is an explanatory view for explaining an internal form of the packaging body for packaging the outer cylinders for the prefilled syringe shown in Fig. 27.
Fig. 29 is a front view of the packaging body for packaging the outer cylinders for the prefilled syringe shown in Fig. 27.
Fig. 30 is a plan view of the packaging body for packaging the outer cylinders for the prefilled syringe shown in Fig. 27.
Fig. 31 is an enlarged cross-sectional view taken along a line A-A of Fig. 30.

### BEST MODE FOR CARRYING OUT THE INVENTION

Using embodiments shown in the drawings, description is made on embodiments of an outer cylinder for a prefilled syringe, a piercing tool for the prefilled syringe, the prefilled syringe, and a packaging body for packaging outer cylinders for the prefilled syringe of the present invention.

It is possible to mount a piercing member 6 including a hollow needle 61 having a needle portion 61a at one end thereof and a needle portion 61b at other end thereof and a piercing member body 60 on which the hollow needle 61 is mounted and which has a piercing member side engaging portion 62 on an outer cylinder 2 of the present invention for the prefilled syringe. The outer cylinder 2 for the prefilled syringe has an outer cylinder body 4 which has a distal part 41 having an opening 48 and an outer cylinder closure member 5 which seals the opening 48 of the distal part 41, accommodates a sealing member 10 through which the needle portion 61b of the hollow needle 61 is penetrable, and is mounted on the distal part 41 of the outer cylinder body 4. The outer cylinder closure member 5 has a piercing member mounting portion 50 on which the piercing member 6 is mountable and a regulation portion which regulates the separation and rotation of the outer cylinder closure member 5 mounted on the outer cylinder body 4. The outer cylinder closure member 5 further includes an outer cylinder closure member side engaging portion 53 which engages the piercing member side engaging portion 62 when the outer cylinder 2 is pressed into the piercing member 6 from the side of the outer cylinder closure member 5 and which regulates the separation of the piercing member 6 from the outer cylinder closure member 5, after the outer cylinder closure member side engaging portion engages the piercing member side engaging portion 62.

The prefilled syringe 1 of the present invention is composed of the outer cylinder 2 for the prefilled syringe, a gasket 8 accommodated inside the outer cylinder 2 and being liquid-tightly slidable inside the outer cylinder, and a medical agent 11 accommodated inside a space formed of the outer cylinder 2 and the gasket 8.

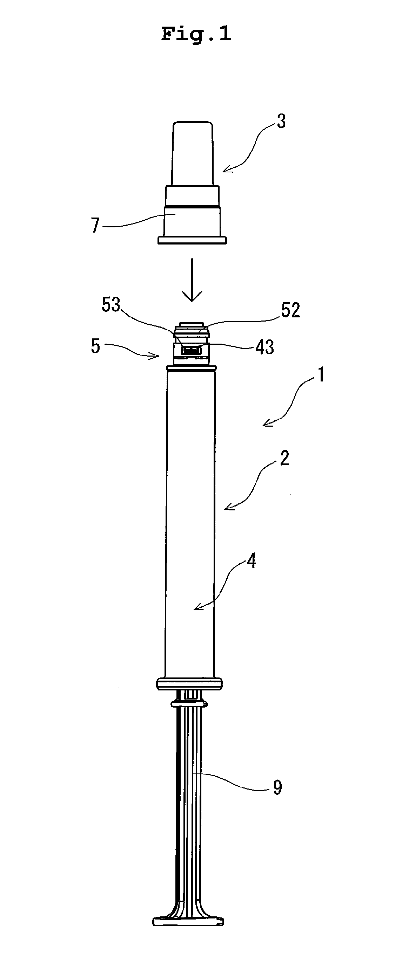

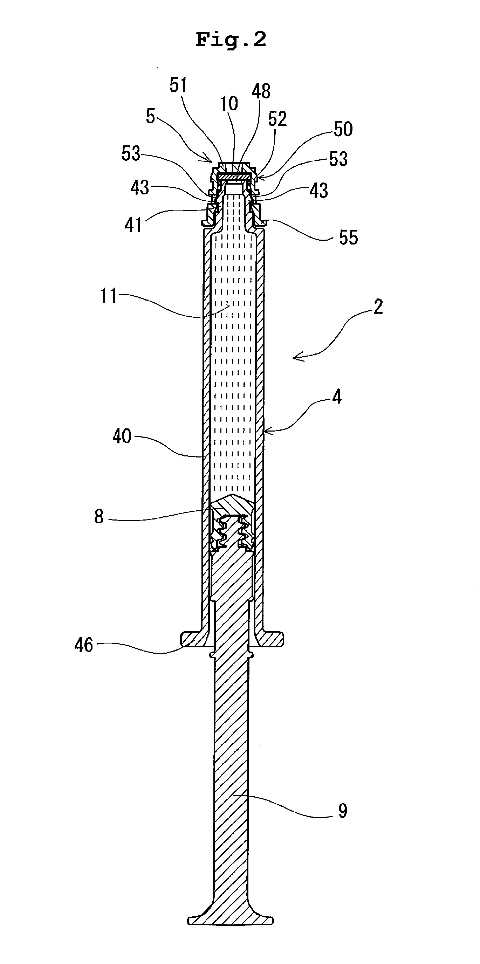

As shown in Figs. 1 and 2, the outer cylinder 2 for the prefilled syringe has the outer cylinder body 4 which has the distal part 41 having the opening 48 and the outer cylinder closure member 5 mounted on the distal part 41 of the outer cylinder body 4.

As shown in Figs. 1, 2, 4 through 7, the outer cylinder body 4 has a body part 40, the distal part 41, and a flange 46. The body part 40 is almost cylindrical and has a liquid accommodation space inside it.

The distal part 41 projects in a distal direction from a shoulder portion of the body part 40. The distal part 41 has the opening 48 for discharging a liquid medicine accommodated inside the outer cylinder and cannot be connected to a medical appliance, having a connection portion to be connected to a nozzle, which conforms to the ISO Standard. That is, the distal part 41 does not have a luer taper configuration unlike an ordinary syringe having the distal portion.

In the outer cylinder body 4 of this embodiment, the distal part 41 has a large-diameter portion 44 formed at a boundary between the distal part 41 and the body part 40, a concave portion 47 (specifically, two opposed concave portions) formed at the large-diameter portion 44, and a projected portion 43 (specifically, opposed two projected portions) formed in the neighborhood of a distal end (in other words, in the neighborhood of a central portion of the distal part 41 in its axial direction) of an intermediate portion 42 (portion having a smaller diameter than the large-diameter portion 44 and a larger diameter than the end of the distal portion) of the distal part 41. The distal part 41 has a small-diameter portion at its distal end. The opening 48 is formed at the distal end of the small-diameter portion.

In the outer cylinder body 4 of this embodiment, the projected portion 43 circumferentially extends a predetermined length. The two concave portions 47 axially extend a predetermined length. The two projected portions 43 and the two concave portions 47 are so formed as not to axially overlap each other. In the outer cylinder, the concave portions 47 and the projected portions 43 are so disposed that one concave portion and the adjacent projected portion form 90 degrees with respect to the central axis of the distal part 41.

The projected portion 43 of the distal part 41 of the outer cylinder body 4 constructs an engaging portion for regulating the separation of the outer cylinder closure member at the outer cylinder side. The concave portion 47 of the distal part 41 constructs an engaging portion for regulating the rotation of the outer cylinder closure member at the outer cylinder side.

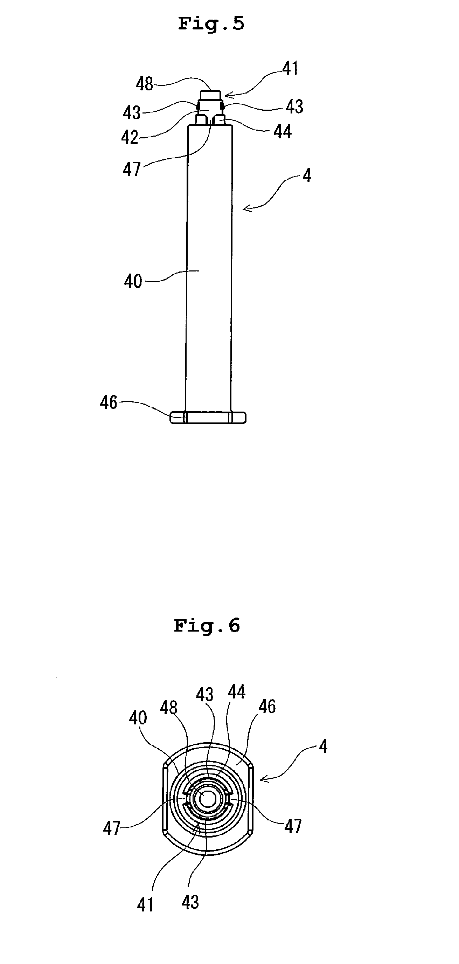

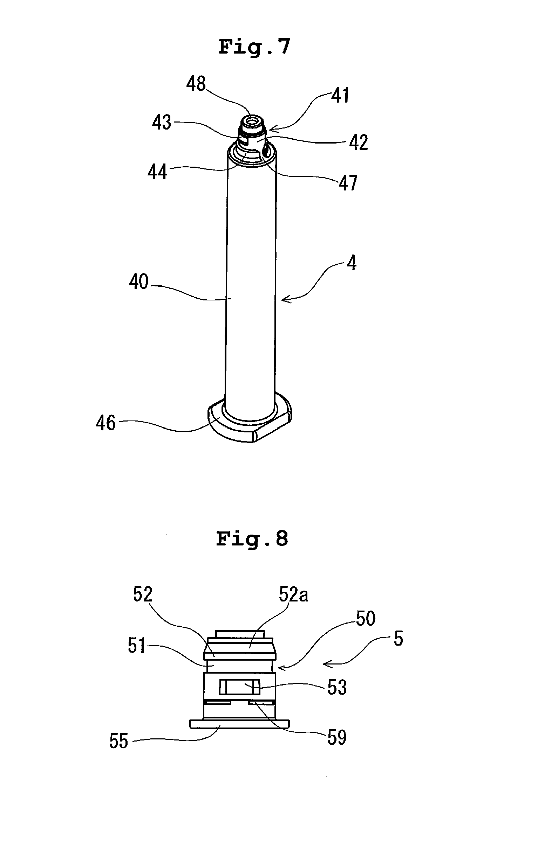

The flange 46 is formed as a disk portion, having the shape of an elliptic donut, which is formed by projecting it from the entire circumference of the rear end of the outer cylinder body 4 in a direction vertical to the outer cylinder body. As shown in Figs. 6 and 7, the flange 46 has two gripping portions opposed to each other.

The outer cylinder body 4 is a tubular body formed of a transparent or semitransparent material which may have oxygen permeability and a low degree of water vapor permeability as necessary.

Examples of a material for the outer cylinder body 4 include resins such as polypropylene, polyethylene, polystyrene, polyamide, polycarbonate, polyvinyl chloride, poly-(4-methylpentene-1), acrylic resin, acrylonitrile-butadiene-styrene copolymer, and polyester including polyethylene terephthalate; and cyclic polyolefin. Of these resins, the polypropylene and the cyclic polyolefin are preferable because these resins can be easily molded and have excellent heat resistance.

The distal part 41 of the outer cylinder body 4 is sealed with the outer cylinder closure member 5 which will be described in detail later.

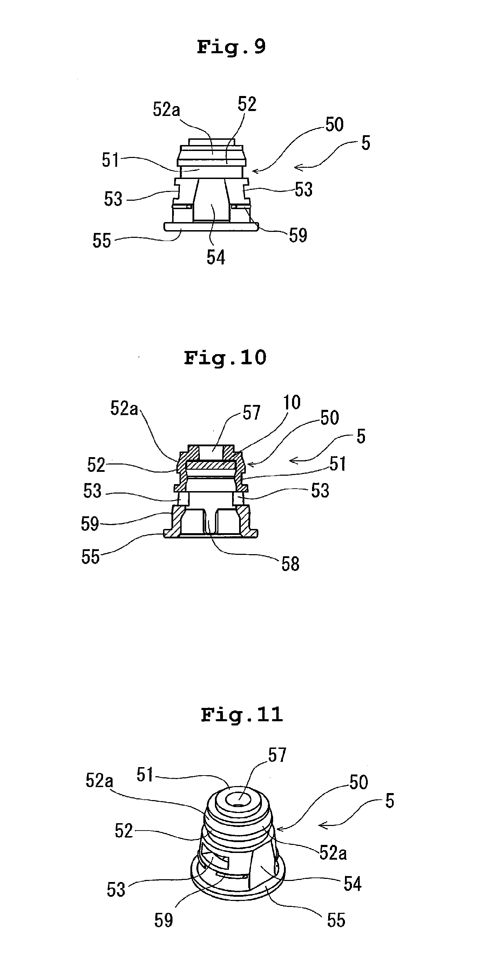

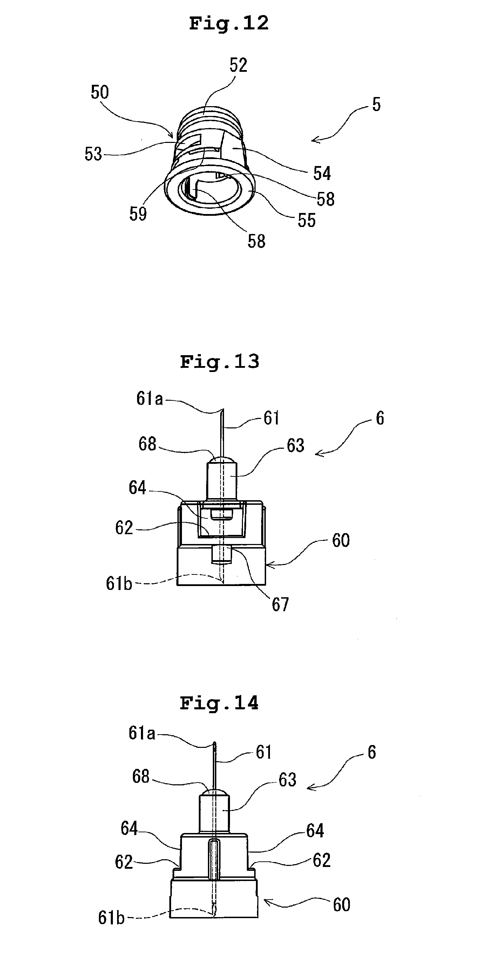

As shown in Figs. 1, 2, 8 through 12, and 22, the outer cylinder closure member 5 has a tubular body part 51 having the piercing member mounting portion 50 and the sealing member 10 accommodated inside the body part 51. The sealing member 10 seals the opening formed at the distal portion of the outer cylinder body.

The body part 51 having the piercing member mounting portion 50 is a tubular body and has a small-diameter open portion 57 formed at a distal end of the body part 51, a flange portion 55 formed at a proximal end thereof, a window portion 53 (specifically, opposed two window portions) formed at a side thereof, a piercing member side engaging portion (annular rib) 52 formed on an outer surface thereof disposed at a side distal from the window portion 53, a rib 59 (a plurality of ribs circumferentially formed) formed at a side distal from the flange portion 55 and proximal from the window portion 53, and a projected portion 58 (specifically, opposed two projected portions) formed on an inner surface of the proximal portion of the body part 51 and axially extending.

In the outer cylinder closure member 5 of this embodiment, the window portion 53 accommodates and engages the projected portion 43 of the outer cylinder body 4 and circumferentially extends a predetermined length. The projected portion 43 of the outer cylinder body 4 enters and engages the window portion 53 of the outer cylinder closure member 5. Thereby the outer cylinder closure member is prevented from separating from the outer cylinder body 4 and from rotating. Thus in the prefilled syringe 3 of this embodiment, the outer cylinder closure member 5 is inseparably mounted on the outer cylinder body 4.

In a state where the outer cylinder closure member 5 is mounted on the outer cylinder body 4, the projected portion 58 formed on the inner surface of the proximal portion of the outer cylinder closure member 5 enters and engages the concave portion 47 of the outer cylinder body 4. Thereby the outer cylinder closure member 5 is unrotatable with respect to the outer cylinder body 4. Thus following the rotation of the outer cylinder body 4, the outer cylinder closure member 5 rotates.

Thus the window portion 53 of the outer cylinder closure member 5 constructs the engaging portion for regulating the separation of the outer cylinder closure member at the tubular body side. The projected portion 58 constructs the engaging portion for regulating the rotation of the outer cylinder closure member at the tubular body side.

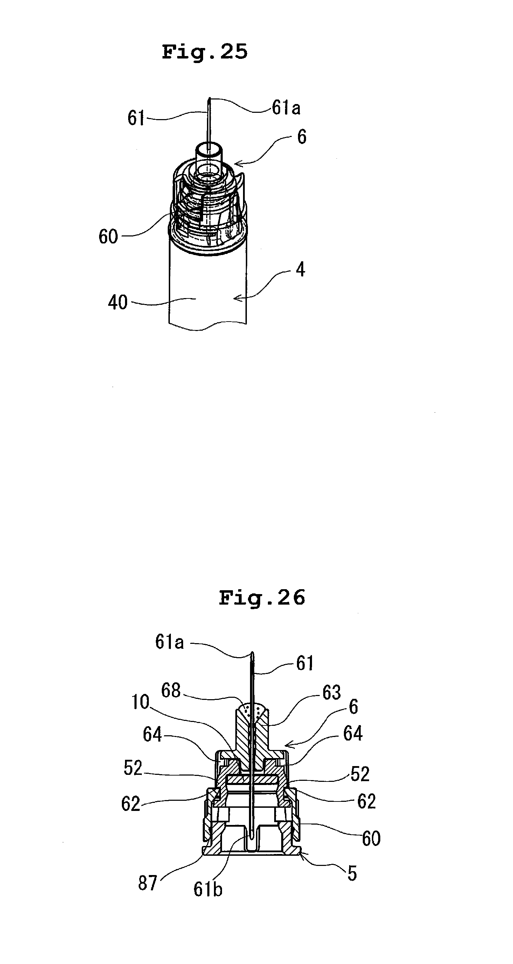

An outer cylinder closure member side engaging portion 52 is formed on a distal side outer surface of the body part 51 having the piercing member mounting portion 50. In this embodiment, the outer cylinder closure member side engaging portion 52 is formed of an annular rib. A distal portion of the annular rib 52 is formed as an annular inclined surface 52a whose diameter decreases toward its distal end. A rear end surface of the annular rib 52 is formed as an erect surface orthogonal to the central axis of the piercing member. As shown in Fig. 26, the outer cylinder closure member side engaging portion 52 engages the piercing member side engaging portion 62 of the piercing member 6 which will be described in detail later.

The outer cylinder closure member 5 of this embodiment has two opposed flat portions 54 on sides disposed at its proximal side to easily grip the outer cylinder closure member 5.

As materials for the outer cylinder closure member, resins such as polypropylene, polyethylene, polystyrene, polyamide, polycarbonate, polyvinyl chloride, poly-(4-methylpentene-1), acrylic resin, acrylonitrile-butadiene-styrene copolymer, and polyester including polyethylene terephthalate; and cyclic polyolefin are listed. Of these resins, the polypropylene and the cyclic polyolefin are preferable because these resins can easily molded and have excellent heat resistance.

The sealing member 10 is accommodated inside the distal portion of the body part 51 having the piercing member mounting portion 50 and seals the open portion 57 and liquid-tightly seals the opening 48 formed at the distal part 41 of the outer cylinder body 4 in a state in which the outer cylinder closure member 5 is mounted on the distal part 41 of the outer cylinder body 4, as shown in Fig. 2. The sealing member 10 is formed of an elastic member capable of liquid-tightly sealing the opening formed at the distal part 41 of the outer cylinder body 4 and allowing the sealing member piercing needle portion 61b of the piercing member 6 to pierce therethrough. The sealing member 10 may have a projected portion which enters the outer cylinder body 4. By so constructing the sealing member, it is possible to decrease a dead volume formed inside the distal portion of the outer cylinder body 4.

As materials for the sealing member, it is preferable to use natural rubber; synthetic rubber such as isoprene rubber, butadiene rubber, fluororubber, and silicone rubber; and thermoplastic elastomers such as olefin elastomers and styrene elastomers.

As shown in Figs. 1 and 2, the prefilled syringe 1 of the present invention is composed of the outer cylinder 2 for the prefilled syringe on which the outer cylinder closure member 5 is mounted, the gasket 8 accommodated inside the outer cylinder 2 and being liquid-tightly slidable inside the outer cylinder 2, and the medical agent 11 accommodated inside the space formed of the outer cylinder 2 and the gasket 8. The prefilled syringe 1 of this embodiment has a plunger 9 mounted on the gasket.

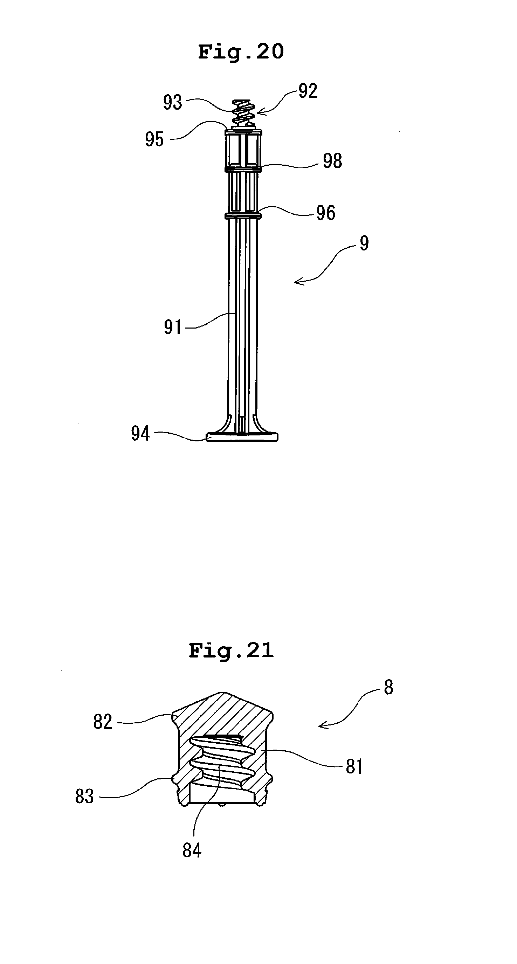

As shown in Figs. 2 and 21, the gasket 8 has a body part 81 which is closed at its distal end and extends in almost an equal diameter and a plurality of annular ribs 82, 83 formed on the body part. The ribs liquid-tightly contact the inner surface of the body part 40 of the outer cylinder body 4.

As materials for the gasket 8, it is preferable to use elastic rubber (for example, butyl rubber, latex rubber, silicone rubber, styrene-butadiene rubber, and isoprene rubber); and thermoplastic elastomers (for example, styrene elastomer such as SBS elastomer, SEBS elastomer, and SEPS elastomer; and olefin elastomer such as ethylene-α-olefin copolymer elastomer).

The gasket 8 has a concave portion extending into the inside thereof from the rear end thereof. The concave portion serves as a plunger mounting portion 84. More specifically, the plunger mounting portion 84 is female screw-like and thus engageable with a male screw portion 93 formed on an outer surface of a gasket-mounting portion 92 formed at a distal portion of the plunger 9. The plunger 9 is mounted on the gasket 8 by the engagement between the female screw portion and the male screw portion.

As shown in Fig. 20, the plunger 9 has the gasket mounting portion 92 formed at a distal portion thereof, a body part 91 cross-shaped in a cross section and axially extending, a flat plate portion 95 provided at a distal end of its body part, a pressing portion 94 provided at a rear end portion of the body part 91, and reinforcing ribs 96, 98 formed halfway on the body part 91. In the plunger of this embodiment, the male screw portion 93 which engages the plunger mounting portion 84 is formed on the outer surface of the gasket mounting portion 92. The plunger 9 is connected to the gasket 8 by the engagement between the plunger mounting portion 84 and the male screw portion 93 of the gasket mounting portion 92. It is possible to use the plunger 9 of a type which is not attached to the gasket in advance but attached thereto when the prefilled syringe is used.

Although the medical agent 11 to be filled inside the outer cylinder body 4 is not limited to a specific one, examples of medical agents include protein preparations such as vitamin preparation (multivitamin preparation), amino acids, antithrombotic agents such as heparin, insulin, antibiotic, antitumor agent, painkillers, cardiotonic drug, intravenous anesthetic, antiperkinsonian agent, anti-ulcer agent, corticosteroid, an antiarrhythmic agent, a hormone drug, an interferon preparation, human immunoglobulin preparation, a blood coagulation factor preparation, and vaccine preparation.

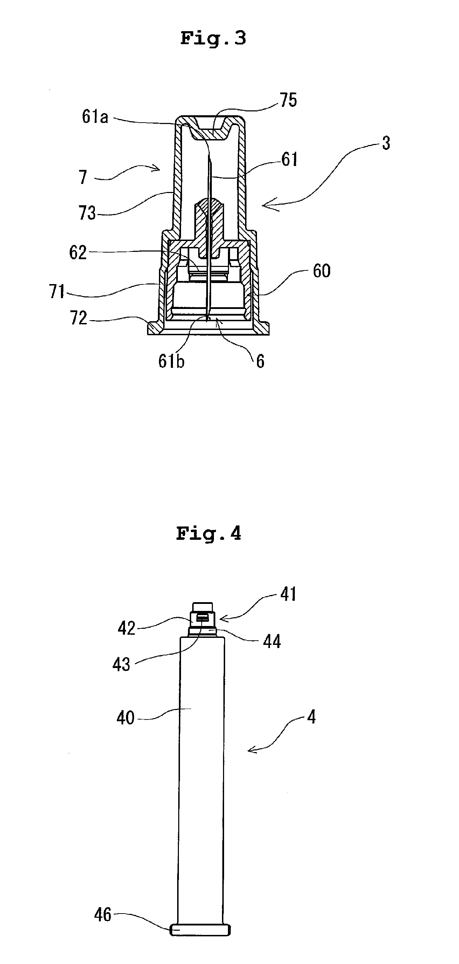

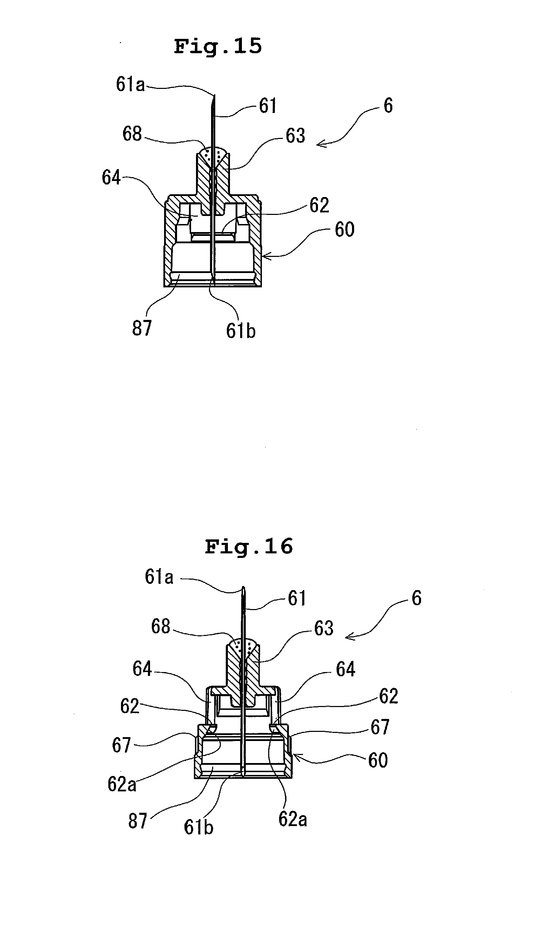

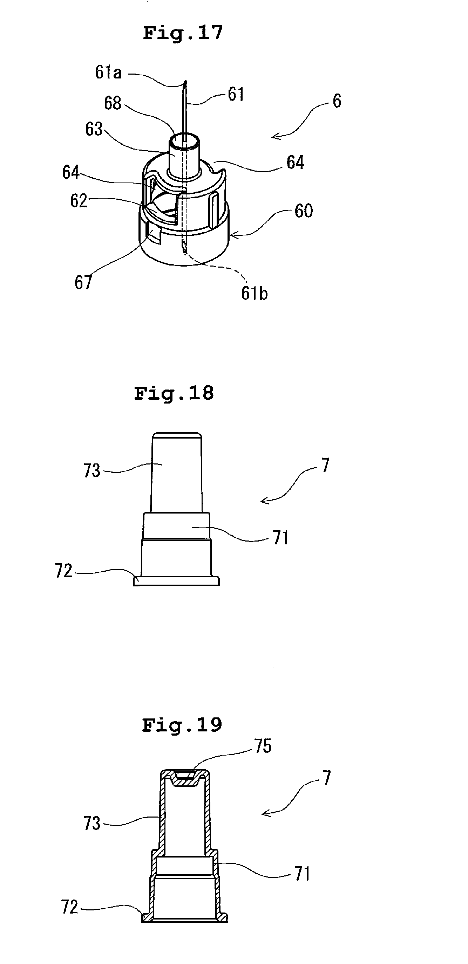

The piercing tool 3 of the present invention for the prefilled syringe is described below with reference to Figs. 3,13 through 19.

The piercing tool 3 of the present invention for the prefilled syringe is used by mounting the piercing tool 3 on the outer cylinder 2 for the prefilled syringe or on the outer cylinder closure member 5 of the prefilled syringe 1. The piercing tool 3 has the hollow needle 61 having the needle portion (specifically, a piercing needle portion 61a at one end thereof, and a sealing member piercing needle portion 61b at the other end thereof) at both ends thereof; the piercing member 6 on which the hollow needle 61 is mounted and which is mountable on the outer cylinder closure member 5 and has the piercing member body 60 having the piercing member side engaging portion 62; and an accommodation member 7 separably accommodating the piercing member 6. The accommodation member 7 is pulled in a distal direction after the piercing member 6 is mounted on the outer cylinder closure member 5, and the piercing member side engaging portion 62 and the outer cylinder closure member side engaging portion 52 engage each other. Thereby the accommodation member 7 is separated from the piercing member 6.

In this embodiment, the outer cylinder closure member side engaging portion is composed of the annular rib 52 formed on the outer surface of the outer cylinder closure member 5. The piercing member side engaging portion is composed of the projection portion 62, formed on the inner surface of the piercing member body 60, which is capable of riding across the annular rib 52 when the outer cylinder closure member 5 is pressed into the piercing member 6.

The piercing tool 3 is composed of the accommodation member 7 and the piercing member 6 accommodated inside the accommodation member 7.

As shown in Figs. 3, 18, and 19, the accommodation member 7 is closed at its one end and cap-shaped. The accommodation member 7 has a tubular body part 71, a closed portion 75 formed at a distal end portion thereof, a small-diameter portion 73 formed at a distal portion of the body part 71, and a flange 72 formed at a rear end portion thereof. The accommodation member 7 accommodates the entire piercing member 6. The accommodation member accommodates the piercing member to prevent a blade face of the hollow needle of the piercing member to be exposed.

As shown in Figs. 3 and 13 through 17, the piercing member 6 has the piercing member body 60 and the hollow needle 61 fixed to the piercing member body 60.

The piercing member body 60 is a tubular body which has a hollow needle fixing part having a small diameter at its distal end portion and an outer cylinder closure member accommodation part at its proximal portion. The outer cylinder closure member accommodation part has an outer cylinder closure member mounting portion on which the outer cylinder closure member 5 of the outer cylinder 2 is mountable.

More specifically, the outer cylinder closure member mounting portion has the piercing member side engaging portion 62 which engages the outer cylinder closure member side engaging portion 52 formed on the outer surface of the outer cylinder closure member 5.

In this embodiment, the piercing member 6 has an open portion 64 (specifically, opposed two open portions). The piercing member side engaging portion is formed of the projection portion 62 projecting into the piercing member 6 from a bottom portion of the open portion 64. The projection portion 62 extends a predetermined length in the circumferential direction of the piercing member body. The form of the piercing member side engaging portion is not limited to a short rib as described above, but may be an annular rib or composed of a plurality of annularly disposed ribs. A lower surface of the projection portion 62 is formed as an inclined surface 62a extended toward the inner surface of the piercing member body. When the piercing member 6 is mounted on the outer cylinder closure member 5, the inclined surface 62a contacts the annular inclined surface 52a of the annular rib 52 of the outer cylinder closure member 5. Because both are formed as the inclined surface, the projection portion 62 is guided in riding across the annular rib 52. After the projection portion 62 rides across the annular rib 52, a rear end surface of the annular rib 52 which is erect and a front end surface of the projection portion 62 which is also erect contact each other. Thereby the piercing member 6 is prevented from separating from the outer cylinder closure member 5.

The piercing member 6 of this embodiment has an annular concave portion 87 formed on an inner surface of the piercing member body in the neighborhood of the proximal portion thereof. When the piercing member 6 is mounted on the outer cylinder closure member 5, the rib 59 (a plurality of ribs circumferentially formed) of the outer cylinder closure member 5 enters the annular concave portion 87, thus engaging the annular concave portion 87. Thereby the piercing member 6 is prevented from separating from the outer cylinder body 4.

Thus the outer cylinder closure member 5 of this embodiment has the first outer cylinder closure member side engaging portion 52 and the second outer cylinder closure member side engaging portion 59 (arc-shaped rib) positioned nearer to the proximal side of the outer cylinder closure member 5 than the first outer cylinder closure member side engaging portion 52. The piercing member 6 has the first piercing member side engaging portion 62 which engages the first outer cylinder closure member side engaging portion 52 and the second piercing member side engaging portion (annular concave portion) 87 positioned at the proximal side of the piercing member 6 and engaging the second outer cylinder closure member side engaging portion 59. The piercing member 6 has a concave portion 67 (specifically, two opposed concave portions) on the outer surface of the piercing member body 60 at an axial central portion thereof.

The hollow needle 61 is fixed to a hollow needle fixing part 63 of the piercing member body 60 with a fixing agent 68 and has the needle portion at both ends thereof. More specifically, one end side of the hollow needle 61 projects to a side distal from the fixing part 63 and has the piercing needle portion 61a to be pierced into a living body at its distal end. The other end side of the hollow needle 61 projects from the fixing portion 63 into the inside of the piercing member 6 and has the sealing member piercing needle portion 61b which can be pierced through the sealing member of the outer cylinder closure member 5 at the end of the other side of the hollow needle 61. The hollow needle 61 extends almost parallel with the central axis of the piercing member body 60.

In the prefilled syringe of this embodiment and an injector for the prefilled syringe, the piercing member 6 mounted on the outer cylinder closure member 5 of the outer cylinder 2 is substantially inseparable from the outer cylinder closure member 5.

In the outer cylinder 2 for the prefilled syringe of this embodiment and the piercing tool 3 therefor, the outer cylinder closure member side engaging portion 52 and the piercing member side engaging portion 62 are so disposed that after the sealing member piercing needle portion 61b of the hollow needle 61 pierces through the sealing member 10, the outer cylinder closure member side engaging portion 52 and the piercing member side engaging portion 62 engage each other.

It is preferable that the outer cylinder 2 of the present invention for the prefilled syringe is subjected to autoclave sterilization. The outer cylinder 2 for the prefilled syringe may be subjected to the autoclave sterilization before it is used. Similarly the prefilled syringe 1 may be subjected to the autoclave sterilization before it is used.

The action of the prefilled syringe 1 of the present invention is described below with reference to Figs. 1 through 3 and 22 through 26.
Figs. 1 through 3 show a state where in the prefilled syringe 1 of the present invention, the outer cylinder closure member 5 is mounted on the distal portion of the outer cylinder 2 and sealed; the piercing member 6 of the piercing tool 3 is accommodated inside the accommodation member 7; and the piercing tool 3 is not mounted on the outer cylinder 2.

As shown in Fig. 2, the outer cylinder closure member 5 is unrotatably and inseparably mounted on the distal part 41 of the outer cylinder body 4. The piercing member 6 is accommodated inside the accommodation member 7.

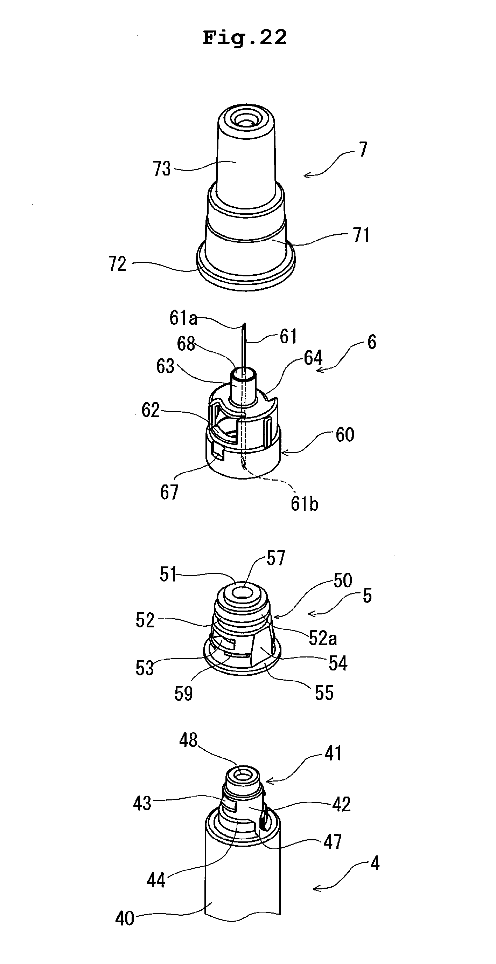

As shown in Fig. 22, the distal part of the outer cylinder 2 of the present invention for the prefilled syringe is composed of the distal part 41 of the outer cylinder body 4, the outer cylinder closure member 5, the piercing member 6, and the accommodation member 7.

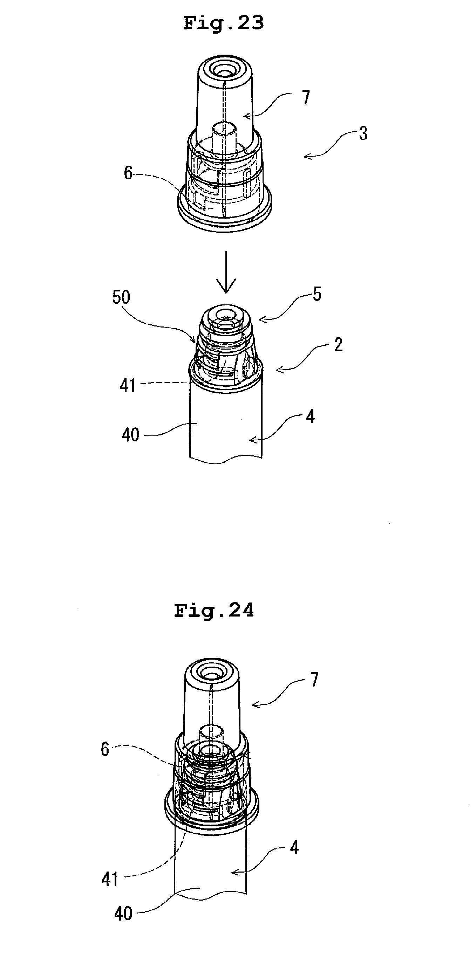

As shown in Fig. 23, when the prefilled syringe is used, the piercing tool 3 is pressed into the outer cylinder 2. Thereby the sealing member piercing needle portion 61b of the hollow needle 61 of the piercing member 6 pierces through the sealing member 10 of the outer cylinder closure member 5. Thereafter the inclined surface 52a of the outer cylinder closure member side engaging portion 52 and the piercing member side engaging portion 62 contact each other. Thereafter by pressing the piercing tool 3 into the outer cylinder 2, the inclined surface 62a of the piercing member side engaging portion 62 advances along the inclined surface 52a of the outer cylinder closure member side engaging portion 52. Thereafter the piercing member side engaging portion 62 rides across the outer cylinder closure member side engaging portion 52. As a result, the outer cylinder closure member side engaging portion 52 and the piercing member side engaging portion 62 engage each other. In a state where the outer cylinder closure member side engaging portion 52 and the piercing member side engaging portion 62 engage each other, the erect surface of the outer cylinder closure member side engaging portion 52 and the piercing member side engaging portion 62 contact each other. Thereby the piercing member 6 is prevented from separating from the outer cylinder closure member 5 (outer cylinder 2). Because an operator has resistance to some extent in the above-described engagement, the operator recognizes that the mounting operation has finished. In the prefilled syringe 1 of this embodiment, simultaneously with or slightly before the above-described engagement, the rib 59 (a plurality of ribs circumferentially formed) of the outer cylinder closure member 5 enters and engages the annular concave portion 87 formed on the inner surface of the piercing member 6 in the neighborhood of its proximal portion. The operator also feels resistance to some extent in this engagement. Because the prefilled syringe has the two engaging mechanisms, the operator securely recognizes that the mounting operation has finished. Fig. 24 shows a state in which the mounting of the piercing tool 3 on the outer cylinder 2 has finished. By pulling the accommodation member 7 in this state, the accommodation member 7 separates from the piercing member 6. As a result, as shown in Fig. 25, the piercing member 6 and the piercing needle part 61b are exposed. In this manner, the preparation for administering a medical agent to a patient finishes. In the prefilled syringe of this embodiment, because the piercing member is mounted on the outer cylinder closure member 5 with the piercing member engaging the syringe body (outer cylinder for prefilled syringe), unintended loosening does not occur.

After an administering operation finishes, the accommodation member 7 is mounted on the syringe body and the syringe is discarded.

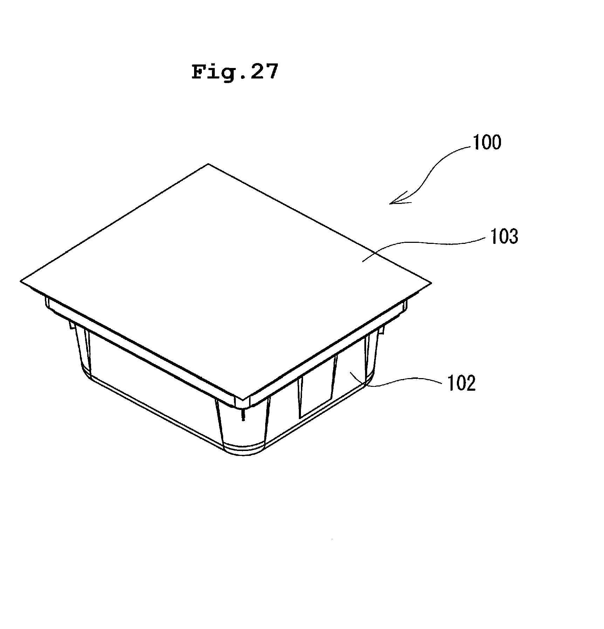

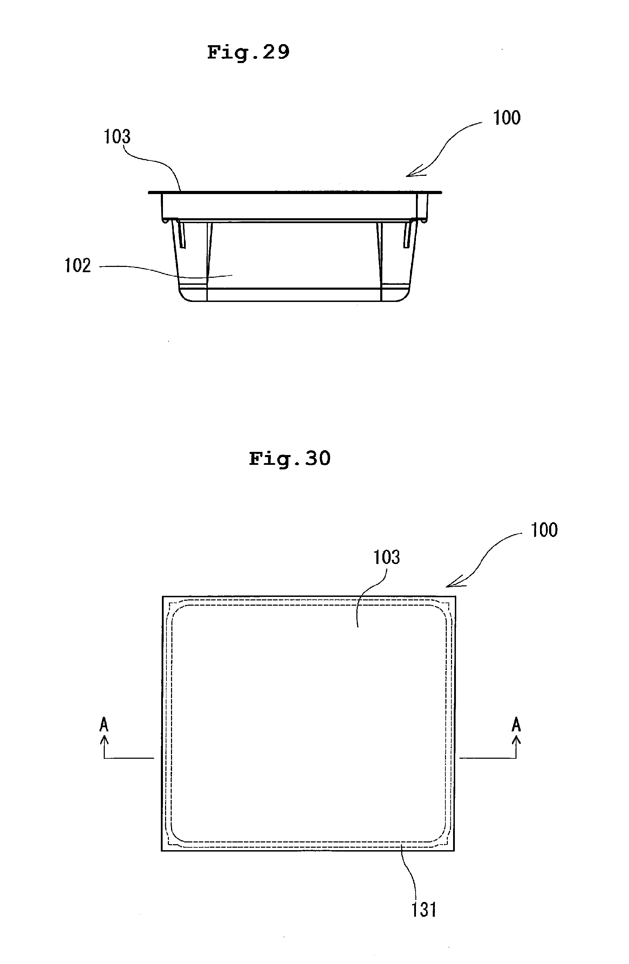

An embodiment of a packaging body of the present invention accommodating a plurality of the outer cylinders for the prefilled syringe is described below with reference to Figs. 27 through 31.

A packaging body 100 of the present invention accommodating a plurality of the outer cylinders for the prefilled syringe can be or is subjected to autoclave sterilization before it is used.

The packaging body 100 has a container 102 whose upper surface is open and which has shape retainability, an outer cylinder holding member 104 capable of holding a plurality of the outer cylinders 2 for the prefilled syringe, a plurality of the outer cylinders 2 for the prefilled syringe held by the outer cylinder holding member 104, and a sheet-shaped lid member 103 which airtightly seals an open upper surface of the container 102 and is peelable from the container 102. The packaging body 100 further includes an air-permeable part, having bacteria impermeability and water vapor permeability, which is provided in the container 102 or the lid member 103.

As shown in Figs. 27 through 31, the packaging body 100 of the present invention for packaging the outer cylinders for the prefilled syringe is composed of the container 102, the outer cylinder holding member 104 capable of holding a plurality of the outer cylinders 2 for the prefilled syringe, a plurality of the outer cylinders 2 for the prefilled syringe held by the outer cylinder holding member 104, and the sheet-shaped lid member 103 which airtightly seals the open upper surface of the container 102 and is peelable from the container 102.

As shown in Figs. 27 through 31, the container 102 has the shape of a tray having strength and shape retainability to some extent and a predetermined depth. The container 102 has a body part 121, an outer cylinder holding member holding part 126, formed at an upper portion of the body part 121, for holding a peripheral portion of the outer cylinder holding member 104 holding the outer cylinders for the prefilled syringe, and an annular flange 124 formed on the periphery of the open upper surface of the container 102.

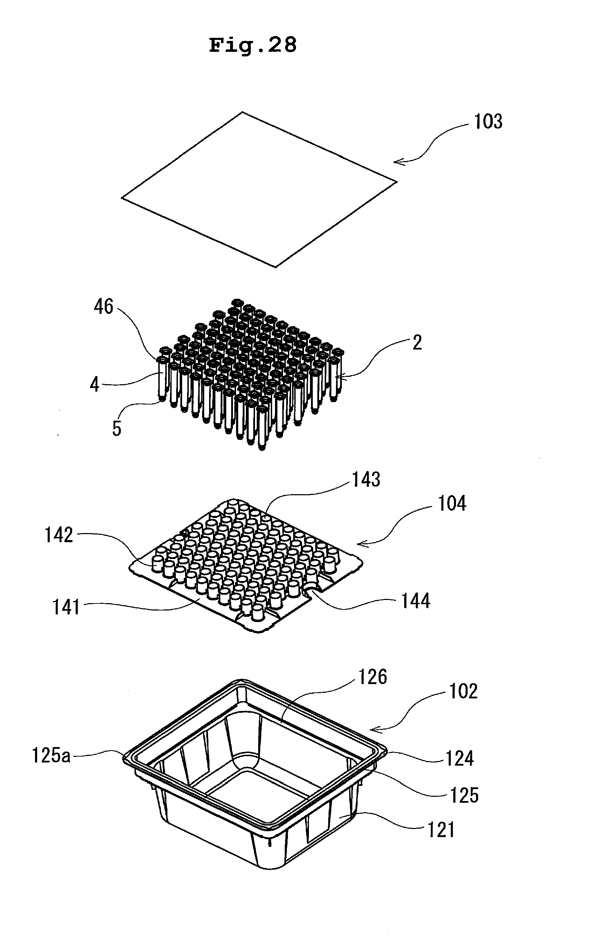

As shown in Figs. 27 and 28, the container 102 of this embodiment is rectangular and bottomed. The thin annular flange 124 is annularly formed on the periphery of the open upper surface of the container 102 by extending the annular flange 124 outward from the periphery of the open upper surface of the container. An annular heat-sealing convex portion 125 is formed on the upper surface of the annular flange 124 to fix the sheet-shaped lid member 103 thereto. The annular heat-sealing convex portion 125 is so formed as to be entirely disposed inward from an outer edge of the annular flange portion and outward from an inner edge of the annular flange portion. A projected portion 125a extending toward the corner of the annular flange 124 is formed at each corner of the annular heat-sealing convex portion 125.

The outer cylinder holding member holding part 126 is formed at a position located below the flange 124 by spacing it at a predetermined interval from the flange 124. In the container 102 of this embodiment, the outer cylinder holding member holding part 126 is formed as an annular stepped portion so that the peripheral portion of the outer cylinder holding member 104 holding the outer cylinders for the prefilled syringe can be placed thereon. The outer cylinder holding member holding part may be annularly uncontinuously formed instead of being annularly continuously formed.

One air-permeable sealing member forming the lid member is used for the container 102 of this embodiment. To do so is preferable because it is easy to perform an operation of fixing the lid member to the container 102.

It is preferable that the container 102 has shape retainablity and rigidity to some extent. To respond to autoclave sterilization, it is desirable to use a thermoplastic material having heat resistance (not less than 120 degrees C) for the container. As materials shape-retainable and rigid to some extent, heat-resistant, and thermoplastic, polyolefin such as polypropylene and polyethylene; vinyl chloride resin, polystyrene/polypropylene resin, polyethylene/ionomer (for example, ethylene-based, styrene-based, fluorine-based)/polyethylene, polyester resin (for example, polyethylene terephthalate, polybutylene terephthalate, and amorphous polyethylene terephthalate), and PP/EVOH/PP (laminate) are listed. In this case, the thickness of the container 102 is favorably 0.05 to 4.00mm and especially favorably 1.00 to 2.00mm.

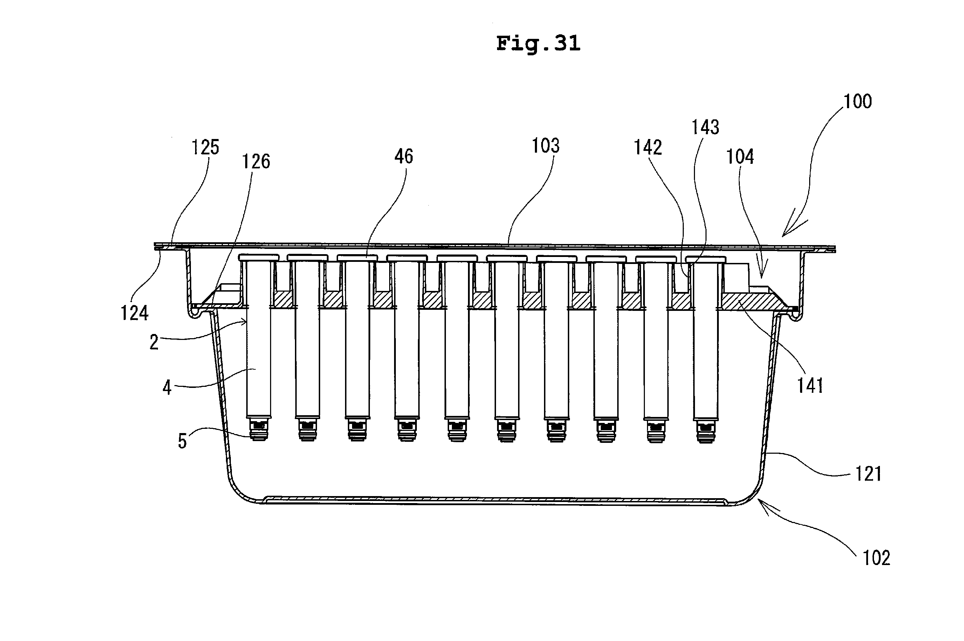

As shown in Figs. 28 and 31, the outer cylinder holding member 104 capable of holding a plurality of the outer cylinders 2 for the prefilled syringe has a substrate part 141 and a plurality of tubular parts 142 projecting upward from the substrate part 141. An opening 143 is formed at the upper end of each tubular part 142. A gripping cut-out part 144 is formed on sides of the substrate part 141. The inner diameter of the tubular part 142 and that of the opening 143 are set larger than the maximum outer diameter portion of the outer cylinder 2 for the prefilled syringe held by the outer cylinder holding member. It is impossible for the flange portion of the outer cylinder 2 for the prefilled syringe held by the outer cylinder holding member to pass through the tubular part 142 and the opening 143. Therefore as shown in Fig. 31, the outer cylinder body 4 of the outer cylinder 2 for the prefilled syringe and the outer cylinder closure member 5 pass through the tubular part 142, but the flange 46 is incapable of passing therethrough. Thus the flange of the outer cylinder is hung at the opening 143 of the tubular part 142. As shown in Fig. 31, the lower end (the distal end of the outer cylinder holding member) of the outer cylinder 2 for the prefilled syringe held by the outer cylinder holding member 104 does not contact the bottom surface of the container 102. It is desirable that materials for forming the outer cylinder holding member 104 have heat resistance (not less than 120 degrees C) so that the materials withstand autoclave sterilization.

As the outer cylinder 2 for the prefilled syringe, the above-described outer cylinder is used.

The sheet-shaped lid member 103 covers and seals the open upper surface of the container 102 and is peelable from the container 102. In the packaging body 100, the sheet-shaped lid member 103 is peelably fixed to the periphery of the open upper surface of the container 102 through an annular heat-sealing portion 131. The sheet-shaped lid member 103 does not substantially permeate bacteria and water vapor therethrough and can be peelably sealed to the container 102.

It is preferable to form the sheet-shaped lid member 103 of a material which does not allow fine particles such as bacteria and virus to permeate therethrough and has water vapor permeability. It is preferable that the sheet-shaped lid member 103 can be heat-sealed to the container 102. For example, it is possible to suitably use nonwoven cloth made of synthetic resin, for example, nonwoven cloth, known as Tyvek (registered trademark), which is made of a synthetic resin material such as polyolefin and a porous film made of synthetic resin.

The peripheral portion of the sheet-shaped lid member 103 is peelably heat-sealed to the heat-sealing convex part 125 formed on the annular flange 124 of the container 102. In this embodiment, the outer edge of the sheet-shaped lid member is not heat-sealed to the annular flange 124 of the container 102 to easily peel the sheet-shaped lid member from the container. The projected portion 125a formed at each corner of the heat-sealing convex part 125 functions as a peeling starting portion. The thickness of the sheet-shaped lid member 103 is favorably 0.05 to 1.00mm and more favorably 0.10 to 0.50mm.

In the above-described embodiment, the lid member has the air-permeable part. But the lid member does not necessarily have the air-permeable part, but the container 102 may have the air-permeable part.

### INDUSTRIAL APPLICABILITY

An outer cylinder for a prefilled syringe of this invention is the following.
(1) An outer cylinder for a prefilled syringe on which a piercing member including a hollow needle having a needle portion at both ends thereof and a piercing member body on which said hollow needle is mounted and which has a piercing member side engaging portion, said outer cylinder for said prefilled syringe has an outer cylinder body which has a distal part having an opening and an outer cylinder closure member which seals said opening of said distal part, accommodates a sealing member through which said needle portion of said hollow needle is penetrable, and is mounted on said distal part of said outer cylinder body; said outer cylinder closure member has a piercing member mounting portion on which said piercing member is mountable and a regulation portion which regulates separation and rotation of said outer cylinder closure member mounted on said outer cylinder body; and said outer cylinder closure member further includes an outer cylinder closure member side engaging portion which engages said piercing member side engaging portion when said outer cylinder for said prefilled syringe is pressed into said piercing member from a side of said outer cylinder closure member and regulates separation of said piercing member from said outer cylinder closure member after said outer cylinder closure member side engaging portion engages said piercing member side engaging portion.

In the outer cylinder for the prefilled syringe, it is easy to perform the operation of mounting the piercing member having the double-ended needle on the outer cylinder closure member of the outer cylinder for the syringe. In addition, in performing the operation of mounting the piercing member on the outer cylinder closure member, the outer cylinder closure member mounted on the outer cylinder for the prefilled syringe does not loosen. Thereby there does not occur loosening-caused leak of the medical liquid. Because the piercing member can be mounted on the syringe in a simple way, the present invention provides the appliance easy to use irrespective of the degree of skill of a user.

Because the piercing member does not separate from the syringe in normal use of the prefilled syringe, the present invention provides an effect of preventing reuse of the piercing member. Thereby the effect of preventing infection to patients and medical care workers through the piercing member is expected.

Further in the case where the piercing needle portion whose distal end is narrow is selected, it is difficult to refill a liquid medicine in a state where the piercing member and the plunger are mounted on the outer cylinder and on the gasket respectively. Thus the outer cylinder for the prefilled syringe is discarded by one-time use. To thin the front end of the piercing needle portion has the effect of not only decreasing patient's pain, but also preventing the piercing member from being continuously used.

The embodiments of the outer cylinder for the prefilled syringe may be carried out as described below.
(2) An outer cylinder for a prefilled syringe according to the above (1), wherein as said regulation portion, each of said distal part of said outer cylinder body and said outer cylinder closure member has a rotation regulation engaging portion and a separation regulation engaging portion.
(3) An outer cylinder for a prefilled syringe according to the above (1), wherein said outer cylinder closure member side engaging portion is an annular rib formed on an outer surface of said outer cylinder closure member; and said piercing member side engaging portion is a projection portion, formed on an inner surface of said piercing member body, which is capable of riding across said annular rib when said outer cylinder closure member is pressed into said piercing member.

(4) An outer cylinder for a prefilled syringe according to the above (1), wherein said outer cylinder closure member has a first outer cylinder closure member side engaging portion and a second outer cylinder closure member side engaging portion positioned nearer to a proximal side of said piercing member mounting portion or a distal side thereof than said first outer cylinder closure member side engaging portion; and said piercing member has a first piercing member side engaging portion which engages said first outer cylinder closure member side engaging portion and a second piercing member side engaging portion positioned at a proximal side of said piercing member or a distal side thereof and engaging said second outer cylinder closure member side engaging portion.
(5) An outer cylinder for said prefilled syringe according to the above (1) subjected to autoclave sterilization.

The outer cylinder for the prefilled syringe subjected to autoclave sterilization can be used as it is in producing an aseptic filling machine.

A prefilled syringe of this invention is the following.
(6) A prefilled syringe comprising an outer cylinder for a prefilled syringe according to claim 1, a gasket accommodated inside said outer cylinder and being liquid-tightly slidable inside said outer cylinder, and a medical agent accommodated inside a space formed of said outer cylinder and said gasket.

It is easy to perform the operation of mounting the piercing member having a double-ended needle on the closure member of the outer cylinder for the syringe. In addition, in performing the operation of mounting the piercing member on the outer cylinder closure member, the outer cylinder closure member mounted on the outer cylinder for the prefilled syringe does not loosen. Thereby there does not occur loosening-caused leak of the medical liquid.

A piercing tool for a prefilled syringe of this invention is the following.
(7) A piercing tool for a prefilled syringe which can be mounted on the above outer cylinder for a prefilled syringe or on said outer cylinder closure member of the above prefilled syringe, said piercing tool has a hollow needle having said needle portion at both ends thereof; said piercing member on which said hollow needle is mounted and which is mountable on said outer cylinder closure member and has said piercing member body having said piercing member side engaging portion; and an accommodation member separably accommodating said piercing member; and said accommodation member is separated from said piercing member by pulling said accommodation member in a distal direction after said piercing member is mounted on said outer cylinder closure member, and said piercing member side engaging portion and said outer cylinder closure member side engaging portion engage each other.

It is easy to perform the operation of mounting the piercing tool on the outer cylinder closure member of the outer cylinder for the syringe. In addition, in performing the operation of mounting the piercing member on the outer cylinder closure member, the outer cylinder closure member mounted on the outer cylinder for the prefilled syringe does not loosen. Thereby there does not occur loosening-caused leak of the medical liquid.

A packaging body for packaging outer cylinders for a prefilled syringe of this invention is the following.
(8) A packaging body for packaging outer cylinders for a prefilled syringe which accommodates a plurality of outer cylinders for a prefilled syringe according to the above (1) and can be subjected to or is subjected to autoclave sterilization, said packaging body comprising a container whose upper surface is open and which has shape retainability; an outer cylinder holding member capable of holding a plurality of said outer cylinders for said prefilled syringe; a plurality of said outer cylinders for said prefilled syringe held by said outer cylinder holding member; a sheet-shaped lid member which airtightly seals said open upper surface of said container and is peelable from said container; and an air-permeable part, having bacteria impermeability and water vapor permeability, which is provided in said container or said lid member.

The packaging body for packaging the outer cylinders for the prefilled syringe is capable of providing the outer cylinder for the prefilled syringe having the above-described high effect in a favorable state.

The embodiments of the packaging body for packaging outer cylinders for a prefilled syringe may be carried out as described below.
(9) A packaging body for packaging outer cylinders for a prefilled syringe according to the above (8) subjected to autoclave sterilization.
(10) A packaging body for packaging outer cylinders for a prefilled syringe according to the above (8) or (9), wherein said lid member is entirely or partly formed as an air-permeable part.

## Claims

1. An outer cylinder for a prefilled syringe on which a piercing member including a hollow needle having a needle portion at both ends thereof and a piercing member body on which said hollow needle is mounted and which has a piercing member side engaging portion,
wherein said outer cylinder for said prefilled syringe has an outer cylinder body which has a distal part having an opening and an outer cylinder closure member which seals said opening of said distal part, accommodates a sealing member through which said needle portion of said hollow needle is penetrable, and is mounted on said distal part of said outer cylinder body;
said outer cylinder closure member has a piercing member mounting portion on which said piercing member is mountable and a regulation portion which regulates separation and rotation of said outer cylinder closure member mounted on said outer cylinder body; and
said outer cylinder closure member further includes an outer cylinder closure member side engaging portion which engages said piercing member side engaging portion when said outer cylinder for said prefilled syringe is pressed into said piercing member from a side of said outer cylinder closure member and regulates separation of said piercing member from said outer cylinder closure member after said outer cylinder closure member side engaging portion engages said piercing member side engaging portion.

2. An outer cylinder for a prefilled syringe according to claim 1, wherein as said regulation portion, each of said distal part of said outer cylinder body and said outer cylinder closure member has a rotation regulation engaging portion and a separation regulation engaging portion.

3. An outer cylinder for a prefilled syringe according to claim 1, wherein said outer cylinder closure member side engaging portion is an annular rib formed on an outer surface of said outer cylinder closure member; and said piercing member side engaging portion is a projection portion, formed on an inner surface of said piercing member body, which is capable of riding across said annular rib when said outer cylinder closure member is pressed into said piercing member.

4. An outer cylinder for a prefilled syringe according to claim 1, wherein said outer cylinder closure member has a first outer cylinder closure member side engaging portion and a second outer cylinder closure member side engaging portion positioned nearer to a proximal side of said piercing member mounting portion or a distal side thereof than said first outer cylinder closure member side engaging portion; and said piercing member has a first piercing member side engaging portion which engages said first outer cylinder closure member side engaging portion and a second piercing member side engaging portion positioned at a proximal side of said piercing member or a distal side thereof and engaging said second outer cylinder closure member side engaging portion.

5. An outer cylinder for said prefilled syringe according to claim 1 subjected to autoclave sterilization.

6. A prefilled syringe comprising an outer cylinder for a prefilled syringe according to claim 1, a gasket accommodated inside said outer cylinder and being liquid-tightly slidable inside said outer cylinder, and a medical agent accommodated inside a space formed of said outer cylinder and said gasket.

7. A piercing tool for a prefilled syringe which can be mounted on an outer cylinder for a prefilled syringe according to claim 1 or on said outer cylinder closure member of said prefilled syringe according to claim 6,
wherein said piercing tool has a hollow needle having said needle portion at both ends thereof; said piercing member on which said hollow needle is mounted and which is mountable on said outer cylinder closure member and has said piercing member body having said piercing member side engaging portion; and an accommodation member separably accommodating said piercing member; and
said accommodation member is separated from said piercing member by pulling said accommodation member in a distal direction after said piercing member is mounted on said outer cylinder closure member, and said piercing member side engaging portion and said outer cylinder closure member side engaging portion engage each other.

8. A packaging body for packaging outer cylinders for a prefilled syringe which accommodates a plurality of outer cylinders for a prefilled syringe according to claim 1 and can be subjected to or is subjected to autoclave sterilization,
said packaging body comprising a container whose upper surface is open and which has shape retainability; an outer cylinder holding member capable of holding a plurality of said outer cylinders for said prefilled syringe; a plurality of said outer cylinders for said prefilled syringe held by said outer cylinder holding member; a sheet-shaped lid member which airtightly seals said open upper surface of said container and is peelable from said container; and an air-permeable part, having bacteria impermeability and water vapor permeability, which is provided in said container or said lid member.

9. A packaging body for packaging outer cylinders for a prefilled syringe according to claim 8 subjected to autoclave sterilization.

10. A packaging body for packaging outer cylinders for a prefilled syringe according to claim 8 or 9, wherein said lid member is entirely or partly formed as an air-permeable part.
